# DEMANDE DE BREVET EUROPEEN

(11) **EP 2 578 581 A1**
(43) Date de publication de la demande: **10.04.2013**
(21) Numéro de dépôt: 12187378.0
(22) Date de dépôt: 05.10.2012
(51) Int. Cl.: C07D 403/04, C07F 7/08

(54) **Nouveaux complexes de terres rares et matériaux hybrides organiques luminescents**

(30) Priorité: 06.10.2011 FR 1159006
(71) Demandeur: Université Blaise Pascal Clermont II, 63006 Clermont Ferrand Cedex 1 (FR); Centre National de la Recherche Scientifique, 75794 Paris Cedex 16 (FR); Ecole Nationale Supérieure de Chimie de Clermont Ferrand, ENSCCF, 63174 Aubière Cédex (FR)
(72) Inventeur: Franville, Anne-Christine, 63000 Clermont-Ferrand (FR); Gautier, Arnaud, 63100 Clermont-Ferrand (FR); Canet, Jean-Louis, 63800 COURNON d'AUVERGNE (FR); Mahiou, Rachid, 63100 CLERMONT FERRAND (FR); Boyer, Damien, 63118 CEBAZAT (FR); Deloncle, Rodolphe, 63170 AUBIERE (FR); Deschamps, Jérôme, 63170 AUBIERE (FR); Adumeau Pierre, 63000 CLERMONT-FERRAND (FR)
(74) Mandataire: Sarlin, Laure V.

(57) **Abrégé**

La présente invention concerne le complexe de formule (A): 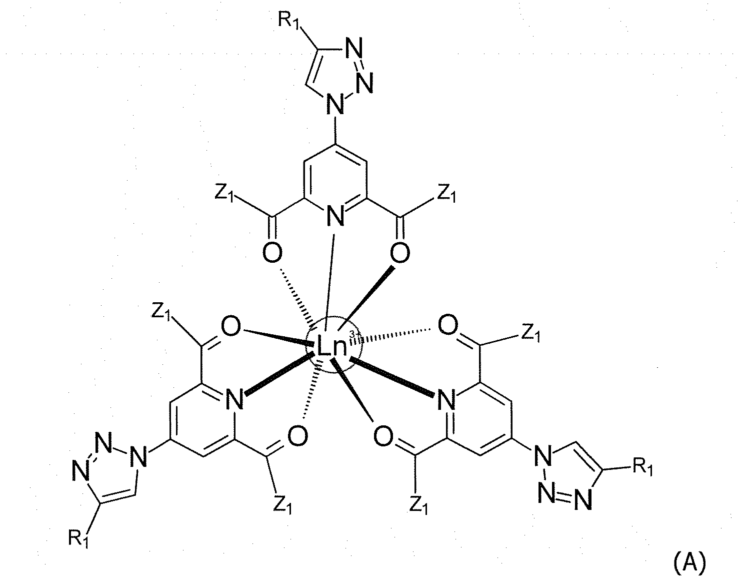 dans laquelle Z₁, R₁ et Ln sont tels que définis à la revendication 1, ainsi que les matériaux formés d'un substrat, fonctionnalisé en surface par greffage covalent de plusieurs complexes (A) identiques ou différents selon l'invention.

## Description

La présente invention concerne le domaine technique des matériaux luminescents. La présente invention concerne, plus particulièrement, des complexes de terre rare, et notamment de lanthanide, fonctionnalisés avec plusieurs fonctions siloxane (type organo-alcoxysilane), les matériaux sur lesquels de tels complexes sont greffés par une liaison O-Si, ainsi que leur procédé de préparation.

Les matériaux luminescents sont utilisés dans de nombreuses applications, en particulier dans le domaine de l'éclairage, de l'affichage, de la traçabilité et notamment du marquage anti-contrefaçon ou en biologie ou dans le secteur solaire photovoltaïque. De nombreux travaux portent donc sur l'élaboration de tels matériaux.

Les marqueurs biologiques classiques de type fluorophores moléculaires souffrent encore d'une durée d'utilisation temporelle limitée par la détérioration, induite par l'environnement, relativement rapide des propriétés optiques (instabilité thermique, oxydation,...). C'est pour cela qu'une nouvelle génération de marqueurs a vu le jour, notamment basée sur des nanoparticules fluorescentes inorganiques pratiquement insensibles au milieu dans lequel elles sont plongées, telles que les nanocristaux de semiconducteurs (Q-dots) dont le représentant le plus connu est le CdS. A côté de leurs avantages indéniables, ces nanoparticules ont des rendements lumineux qui restent faibles comparativement aux fluorophores moléculaires classiques, et la présence du cadmium, métal lourd très toxique, en limite l'usage (les particules sont d'ailleurs souvent recouvertes de ZnS et/ou de SiO₂ matériaux réputés être plus biocompatibles). D'autres marqueurs inorganiques nanoparticulaires, métalliques (Au, Ag) ou oxydes (Ln₂O₃ et LnVO₄ avec Ln = Y, Lu, Gd ou La) activés par Eu³⁺ ou Tb³⁺ sont aussi actuellement utilisés comme traceurs pour l'imagerie dans le suivi médical. Leurs émissivités restent cependant plus faibles que celles des Q-dots et des fluorophores moléculaires. De plus, un des plus gros désavantages de ces systèmes à base d'oxydes de lanthanides est la nécessité d'utiliser des rayonnements de courtes longueurs d'onde pour sensibiliser l'émission de la luminescence.

Un certain nombre de publications antérieures visent également l'encapsulation d'entités luminescentes dans de la silice. Un réenrobage des particules luminescentes permet d'obtenir une structure coeur/coquille dont les effets sont principalement : une augmentation de la photostabilité due à la présence de la matrice de silice qui joue un rôle de barrière, une exaltation de la luminescence par le regroupement de plusieurs molécules fluorescentes par nanoparticule, et une compatibilité accrue de la nanoparticule avec différents milieux notamment biologiques.

D'autres solutions ont également porté sur la complexation de lanthanides par des molécules organiques adaptées pour permettre d'exciter l'émission d'une fluorescence à des énergies plus faibles (proche UV-visible) tout en augmentant le coefficient d'absorption par l'effet dit d'antenne.

A titre d'exemple, on peut citer la demande de brevet EP 2 112 211 qui décrit une particule de silice incluant au moins un complexe d'au moins un lanthanide et d'au moins un ligand comprenant une entité 1,4,7 triazacyclononane. Dans de telles particules, le complexe se trouve encapsulé dans la particule de silice. Il est également décrit, d'une manière générale, que le complexe peut être greffé en surface, sans décrire précisément comment un tel greffage peut être réalisé. Les complexes décrits dans cette demande de brevet ne sont pas adaptés pour élaborer des revêtements greffés par liaison covalente. De plus, les nanoparticules décrites sont des hybrides de classe 1 présentant des interactions dites faibles entre la silice et les complexes organiques. Leur stabilité n'est donc pas satisfaisante.

Certains des inventeurs de la présente demande de brevet ont également décrit dans Dalton Trans. 2010, 39, 7091-7097 des matériaux répondant à la représentation schématique ci-dessous : 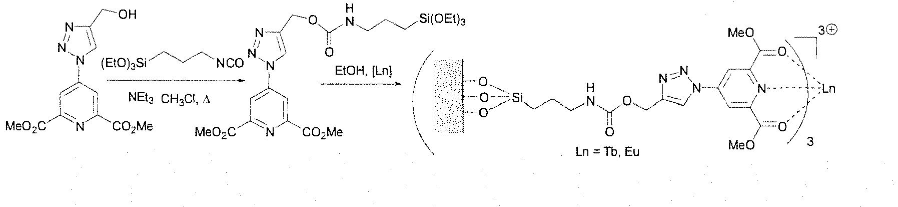 dans lesquels un complexe de terre rare est greffé à la surface d'un support de silice. Les possibilités de fonctionnalisation et/ou de modification du spectre d'absorption sont quasi-inexistantes avec de tels complexes.

Par ailleurs, d'autres travaux de certains des inventeurs de la présente demande de brevet (Solid State Sciences 2001, 3, 211-222) ont aussi porté sur des matrice sol-gel incorporant différents complexes de lanthanides. Le protocole de synthèse n'offre pas de possibilité de fonctionnalisation et/ou de modification du spectre d'absorption des complexes obtenus.

Dans ce contexte, la présente invention se propose de fournir de nouveaux complexes de terre rare, et notamment de lanthanide pouvant être facilement greffés par liaison covalente sur différents substrats.

Un autre objectif de l'invention est de proposer des structures qui puissent être modifiées, de manière à moduler le spectre d'absorption obtenu.

Par ailleurs, la présente invention propose de nouvelles structures qui peuvent, même après greffage, présenter des fonctions réactives libres pour le greffage ultérieur de molécules d'intérêt ou l'encrage de fonction siloxane pour la croissance d'une couche de silice protectrice.

La présente invention a donc pour objet des complexes de formule (A) : 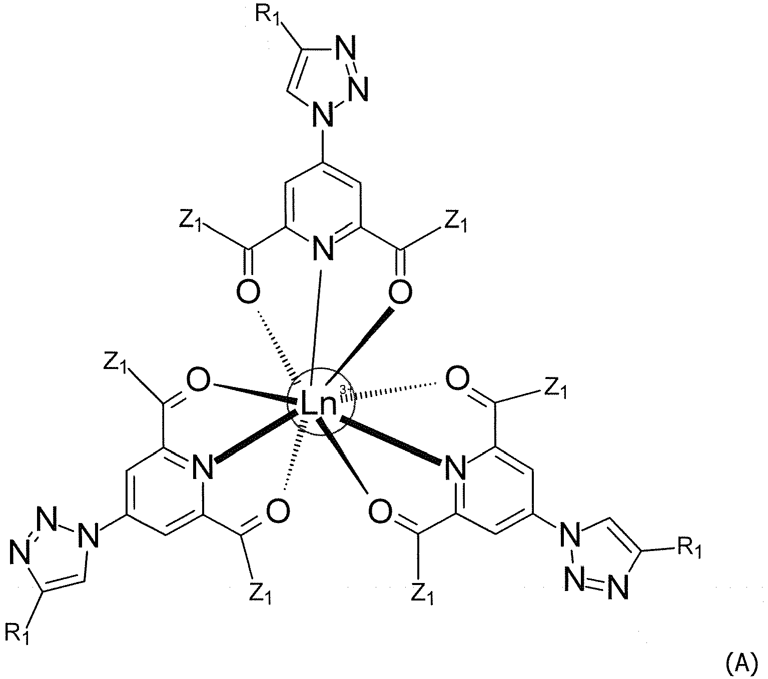 dans laquelle :
- Z₁ et représente -X-L-Si(ORₐ)₃ avec X qui peut être O ou NR₄ avec R₄ qui représente un atome d'hydrogène, un groupe alkyle comprenant de 1 à 4 atomes de carbone ou un groupe -(CH₂)ₓ-Si(OR'ₐ)₃ avec x qui est égal à 1, 2, 3, 4, 5 ou 6 et R'ₐ qui représente un groupe alkyle comprenant de 1 à 4 atomes de carbone; L qui représente une chaine hydrocarbonée, saturée, substituée ou non substituée, comprenant de 1 à 6 atomes de carbone ; et Rₐ qui représente un groupe alkyle comprenant de 1 à 4 atomes de carbone ;
- R₁ et représente un atome d'hydrogène ou un groupe alkyle de 1 à 6 atomes de carbone pouvant comporter un ou plusieurs hétéroatomes, un groupe cycloalkyle de 3 à 7 atomes de carbone pouvant comporter un ou plusieurs hétéroatomes, un groupe aryle ou hétéroaryle, lesdits groupes alkyle, cycloalkyle, aryle et hétéroaryle pouvant être non substitués ou substitués,
- et Ln est une terre rare complexée à un degré d'oxydation 3,

Selon des modes de réalisation particuliers, les complexes selon l'invention présentent l'une ou l'autre des caractéristiques ci-dessous, ou une combinaison des caractéristiques ci-dessous lorsqu'elles ne s'excluent pas l'une l'autre, voire toutes les caractéristiques ci-dessous :
- Z₁ représente -X-L-Si(ORₐ)₃ avec X tel que défini à la revendication 1, L qui représente -(CH₂)_{y}- avec y qui est égale à 1, 2, 3, 4, 5 ou 6, Rₐ qui représente un groupe alkyle comprenant de 1 à 4 atomes de carbone ;
- X représente NR₄, avec R₄ qui représente un atome d'hydrogène, un groupe méthyle, éthyle ou un groupe -(CH₂)ₓ-Si(OR'ₐ)₃ avec R'ₐ qui représente un groupe méthyle ou éthyle et x qui est égal à 1, 2, 3, 4, 5 ou 6; de tels complexes de type diamides sont plus efficaces pour la complexation des ions de terres rares que leurs analogues de type diester ;
- R₁ représente un atome d'hydrogène ou un groupe alkyle de 1 à 6 atomes de carbone, un groupe alkyle de 1 à 6 atomes de carbone interrompu par un groupe amino, un groupe cycloalkyle de 3 à 7 atomes de carbone, un groupe phényle, naphtyle, pyridyle, thiophényle ou imidazolyle, lesdits groupes alkyle, cycloalkyle, phényle, naphtyle, pyridyle, thiophényle et imidazolyle pouvant être non substitués ou substitués par un ou plusieurs substituants choisis parmi :
- les atomes de brome, chlore, iode ou fluor ;
- les alkyles de 1 à 20, et de préférence de 1 à 6 atomes de carbone, et notamment les groupes méthyle, éthyle, n-propyle ou iso-propyle ; lesdits groupes alkyle pouvant être non substitués ou substitués par un groupe -OR₀ avec R₀ qui représente un atome d'hydrogène ou un alkyle de 1 à 20, et de préférence de 1 à 6, atomes de carbone, un aryle ou un groupe aralkyle de 7 à 18 atomes de carbone, et notamment les groupes méthoxy, éthoxy, n-propoxy ou iso-propoxy et phénoxy ;
- les groupes cycloalkyle, et notamment les groupe cyclopentyle, cyclohexyle et cycloheptyle ; lesdits groupes alkyle pouvant être non substitués ou substitués par un groupe -OR₀ avec R₀ qui représente un atome d'hydrogène ou un alkyle de 1 à 20, et de préférence de 1 à 6, atomes de carbone, un aryle ou un groupe aralkyle de 7 à 18 atomes de carbone, et notamment les groupes méthoxy, éthoxy, n-propoxy ou iso-propoxy et phénoxy ;
- les groupes phényle et naphtyle ;
- les groupes nitro (-NO₂) carboxyl (-COOH),
- les groupes -OR₀ avec R₀ qui représente un atome d'hydrogène ou un alkyle de 1 à 20, et de préférence de 1 à 6, atomes de carbone, un aryle ou un groupe aralkyle de 7 à 18 atomes de carbone, et notamment les groupes méthoxy, éthoxy, n-propoxy ou iso-propoxy et phénoxy ;
- les groupes amino -NR₀R'₀ avec R₀ et R'₀, identiques ou différents, qui représentent un atome d'hydrogène ou un alkyle de 1 à 20, et de préférence de 1 à 6, atomes de carbone, un aryle ou un groupe aralkyle de 7 à 18 atomes de carbone, et notamment les groupes -NH₂, -NHMe, - NMe₂, -NHEt, -NEt₂,
- Ln est choisi parmi Y, Gd, Eu, Tb, Yb, Er, Tm, Pr, Ho et Nd.

Les composés appartenant aux trois familles préférées suivantes, qui peuvent répondre, aux modes de réalisations particuliers donnés dans la description précédente, présentent des possibilités d'excitation IR à 2 ou 3 photons et de bon rendement de fluorescence avec des bandes d'excitation qui varient en fonction du choix de R₁ :
1) Famille 1 : R₁ représente un groupe un groupe alkyle de 1 à 6 atomes de carbone, un groupe alkyle de 1 à 6 atomes de carbone interrompu par un groupe amino, un groupe cycloalkyle de 3 à 7 atomes de carbone, lesdits groupes alkyle et cycloalkyle pouvant être non substitués ou substitués par un groupe -OR₀ avec R₀ qui représente un atome d'hydrogène ou un alkyle de 1 à 20, et de préférence de 1 à 6, atomes de carbone, un aryle ou un groupe aralkyle de 7 à 18 atomes de carbone, et notamment un groupe méthoxy, éthoxy, n-propoxy, iso-propoxy ou phénoxy ;
2) Famille 2 : R₁ représente :
   - un groupe phényle, un groupe 4-fluorophényle ou 2-fluorophényle, un groupe phényle substitué par un ou plusieurs groupes alkyle ou cycloalkyle porteurs ou non d'un groupe -OR₀ avec R₀ qui représente un atome d'hydrogène ou un alkyle de 1 à 20, et de préférence de 1 à 6, atomes de carbone, un aryle ou un groupe aralkyle de 7 à 18 atomes de carbone, et notamment un groupe méthoxy, éthoxy, n-propoxy, iso-propoxy ou phénoxy,
   - un groupe 2-pyridyle ou un groupe 2-pyridyle substitué par un ou plusieurs groupes alkyle ou cycloalkyle porteurs ou non d'un groupe -OR₀ avec R₀ qui représente un atome d'hydrogène ou un alkyle de 1 à 20, et de préférence de 1 à 6, atomes de carbone, un aryle ou un groupe aralkyle de 7 à 18 atomes de carbone, et notamment un groupe méthoxy, éthoxy, n-propoxy, iso-propoxy ou phénoxy, ou
   - un groupe 2-thiophényle ou un groupe 2-thiophényle substitué par un ou plusieurs groupes alkyle ou cycloalkyle porteurs ou non d'un groupe -OR₀ avec R₀ qui représente un atome d'hydrogène ou un alkyle de 1 à 20, et de préférence de 1 à 6, atomes de carbone, un aryle ou un groupe aralkyle de 7 à 18 atomes de carbone, et notamment un groupe méthoxy, éthoxy, n-propoxy, iso-propoxy ou phénoxy ;
3) Famille 3 : R₁ représente :
   - un groupe phényle substitué en position 2 ou 4 par -NO₂ ou par un groupe alcoxy de 1 à 20, et de préférence de 1 à 6, atomes de carbone ou un groupe cycloalcoxy de préférence de 3 à 7 atomes de carbone, les partie alkyle et cycloalkyle étant porteuse ou non d'un groupe -OR₀ avec R₀ qui représente un atome d'hydrogène ou un alkyle de 1 à 20, et de préférence de 1 à 6, atomes de carbone, un aryle ou un groupe aralkyle de 7 à 18 atomes de carbone, et notamment un groupe méthoxy, éthoxy, n-propoxy, iso-propoxy ou phénoxy ; ou
   - un groupe 4-imidazolyle ou un groupe 4-imidazolyle substitué par un ou plusieurs groupes alkyle ou cycloalkyle porteurs ou non d'un groupe -OR₀ avec R₀ qui représente un atome d'hydrogène ou un alkyle de 1 à 20, et de préférence de 1 à 6, atomes de carbone, un aryle ou un groupe aralkyle de 7 à 18 atomes de carbone, et notamment un groupe méthoxy, éthoxy, n-propoxy, iso-propoxy ou phénoxy.

De manière préférée dans le cadre de l'invention, le substituant R₁ est différent de l'hydrogène, ce qui permet de moduler l'émission de fluorescence du complexe obtenu.

Dans la définition des complexes ci-dessus, on entend :
Par « alkyle », on entend un groupement hydrocarboné saturé, linéaire ou ramifié, et par exemple un méthyle, éthyle, n-propyle, iso-propyle, ter-butyle, n-butyle, n-heptyle, ou n-hexyle.
Par « cycloalkyle », on entend un groupement hydrocarboné saturé ou insaturé constitué d'au moins un cycle, éventuellement ponté. A titre d'exemple, on peut citer les groupes cyclopropyle, cyclobutyle, cyclopentyle, cyclohexyle, cycloheptyle, adamantyle et cyclohexene.
Par « aryle », on entend un groupe hydrocarboné constitué d'un ou plusieurs cycles aromatiques, comprenant au plus 20 atomes de carbone. A titre d'exemple de groupe aryle préféré, on peut citer le phényle et le naphtyle.
Par « hétéroaryle », on entend un groupe mono ou polyclyclique aromatique de 5 à 14 chainons, de préférence de 5 à 10 chainons et dans lequel au moins un des atomes formant le mono ou le polycycle est différent du carbone et est choisi parmi O, N, S. Les hétéroaryles à 5 ou 6 chainons sont préférés. A titre d'exemple d'hétéroaryle, on peut citer les groupes pyridyle, pyrazinyle, furanyle, thiényle, pyrimidinyle, isoxazolyle, isothiazolyle, oxazolyle, thiazolyle, pyrazolyle, furazanyle, pyrrolyle, pyrazolyle, triazolyle, 1 ,2,4-thiadiazolyle, pyrazinyle, pyridazinyle, quinoxalinyle, phthalazinyle, imidazo[1 ,2-a]pyridinyle, imidazo[2,1-b]thiazolyle, benzofurazanyle, indolyle, azaindolyle, benzimidazolyle, benzothiényle, quinolinyle, imidazolyle, thienopyridyle, quinazolinyle, thiénopyrimidyle, pyrrolopyridyle, imidazopyridyle, isoquinolinyle, benzoazaindolyle, 1 ,2,4-triazinyle, and benzothiazolyle.

Lorsque dans le cadre de l'invention, un groupe est dit substitué, il peut, par exemple, être substitué par un ou plusieurs substituants choisis parmi les atomes de brome, chlore, iode ou fluor ; les alkyles de 1 à 20, et de préférence de 1 à 6, atomes de carbone, substitués ou non substitués et notamment les groupes méthyle, éthyle, n-propyle ou iso-propyle ; les groupes cycloalkyle comportant de préférence de 3 à 7 atomes de carbone, substitués ou non substitués, et notamment les groupe cyclopentyle, cyclohexyle et cycloheptyle ; les groupes phényle et naphtyle substitués ou non substitués ; les groupes nitro (-NO₂), carboxyl (-COOH), les groupes - OR₀ avec R₀ qui représente un atome d'hydrogène ou un alkyle de 1 à 20, et de préférence de 1 à 6, atomes de carbone, substitué ou non substitué, un cycloalkyle comportant de préférence de 3 à 7 atomes de carbone, substitué ou non substitué, un aryle substitué ou non substitué ou un groupe aralkyle de 7 à 18 atomes de carbone substitué ou non substitué, et notamment les groupes méthoxy, éthoxy, n-propoxy ou iso-propoxy et phénoxy ; les groupes amino -NR₀R'₀ avec R₀ et R'₀, identiques ou différents, qui représentent un atome d'hydrogène ou un alkyle de 1 à 20, et de préférence de 1 à 6, atomes de carbone, substitué ou non substitué un aryle substitué ou non substitué ou un groupe aralkyle substitué ou non substitué de 7 à 18 atomes de carbone, et notamment les groupes -NH₂, -NHMe, -NMe₂, NHEt, - NEt₂

Par « aralkyle de 7 à 18 atomes de carbone », on entend un groupe alkyle tel que précédemment défini comprenant de 1 à 8 atomes de carbone substitué par un groupe phényle ou naphtyle.

Les complexes selon l'invention sont préparés à partir des ligands du type : 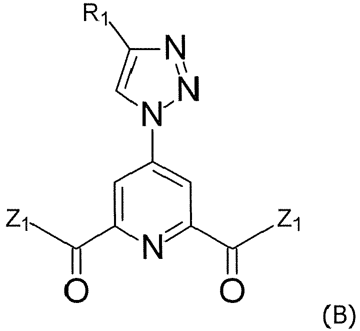 dans lesquels Z₁ et R₁ sont tels que définis précédemment pour les complexes (A). De tels ligands sont préparés selon les techniques détaillées dans les exemples ci-après ou des techniques analogues bien connues de l'homme de l'art.

Le ou les ligands sélectionnés sont mis en solution dans un solvant dans lequel il(s) est (sont) soluble(s), de préférence de l'éthanol ou du méthanol anhydre, avec un sel de la terre rare souhaitée, notamment sous la forme d'un nitrate ou chlorure. De façon avantageuse, 3 équivalents molaires de ligands sont utilisés par atome de terre rare. La complexation peut être réalisée à température ambiante, notamment à une température de 18 à 30 °C , ou au reflux, dans un temps relativement court, par exemple de l'ordre de 10 minutes à 1 heure.

La présente invention a également pour objet les matériaux formés d'un substrat, fonctionnalisé en surface par greffage covalent de plusieurs complexes (A) précédemment définis, identiques, le greffage covalent étant réalisé selon une liaison Si-O par l'intermédiaire d'un des atomes de silicium présent dans Si(ORₐ)₃ au niveau des substituants Z₁ tels que définis pour (A). Dans le cadre de l'invention, il a été mis en évidence que même après greffage, les principales propriétés d'absorption des complexes (A) obtenues grâce au choix de R₁ notamment étaient conservées.

Selon des modes de réalisation particuliers, le matériau selon l'invention présente l'une ou l'autre des caractéristiques ci-dessous, ou une combinaison des caractéristiques ci-dessous lorsqu'elles ne s'excluent pas l'une l'autre, voire toutes les caractéristiques ci-dessous :
- le matériau se présente sous la forme d'un objet massif, d'un film ou de nanoparticules, notamment sous la forme d'une poudre ;
- le substrat est constitué d'un oxyde d'un élément M et la liaison covalente entre le complexe (A) est le substrat est Si-O-M. En particulier, l'élément M est le silicium, ou un métal, par exemple Cu ou AI ;
- le substrat se présente sous la forme de nanoparticules de silice ;
- le substrat est fonctionnalisé en surface par greffage covalent de différents complexes (A) qui diffèrent par la nature de l'ion Ln³⁺ piégé ou par la nature du substituant R₁ ;
- les complexes (A) forment un revêtement avec création de pont -Si-O-Si entre complexes greffés ;
- les complexes greffés en surface du substrat sont recouverts d'une couche de silice, éventuellement fonctionnalisée, par exemple par des fonctions -NH₂, -C≡CH, -COOH, -N₃ ou OH ;
- le substrat est susceptible d'être obtenu par un procédé comprenant les étapes successives de :
   a) Dépôt de complexes de formule (A) ou le plus souvent en solution, sur un substrat sélectionné, présentant en surface des fonctions OH libres,
   b) Traitement thermique pour permettre la formation de liaisons covalentes O-Si entre le substrat et les complexes par l'intermédiaire d'un des atomes de silicium des complexes présent dans Si(ORₐ)₃ d'au moins un des substituants Z₁ tels que définis pour (A), à la place d'au moins certaines des fonctions OH qui étaient présentes en surface du support, voire formation de ponts Si-O-Si entre complexes.

La préparation des matériaux conformes à l'invention peut donc se faire en deux étapes :
a) Dépôt de complexes de formule (A) identiques, le plus souvent en solution, sur un substrat sélectionné, présentant en surface des fonctions OH libres,
b) Traitement thermique pour permettre la formation de liaisons covalentes O-Si entre le substrat et les complexes par l'intermédiaire d'un des atomes de silicium des complexes présent dans Si(ORₐ)₃ d'au moins un des substituants Z₁ tels que définis pour (A), à la place d'au moins certaines des fonctions OH qui étaient présentes en surface du support, voire formation de ponts Si-O-Si entre complexes.

Le ou les complexes peuvent être déposés par différentes techniques telles que le trempage-retrait, l'enduction centrifuge, le spray ou la pulvérisation. En général, le dépôt est réalisé à partir d'une solution du ou des complexes sélectionnés. En général, les organo-alcoxysilanes sont solubilisés dans la quantité nécessaire et suffisante d'éthanol anhydre. Le dépôt peut être réalisé sur différents types de substrats à condition que la surface présente des fonctions hydroxyles permettant la formation de liaisons siloxane avec le complexe organométallique, et en particulier avec ses parties Si(ORₐ)₃ avec Rₐ tel que défini pour (A). A titre d'exemple, de substrats, on peut citer les substrats de verre, Pyrex, quartz, les tissus, les polymères, métaux traités en surface, la silice, sous la forme de support plan ou usiné, mais aussi de forme complexe, ou de particules et de nanoparticules notamment. Par nanoparticules, on entend des particules, et de préférence des particules essentiellement sphériques, dont la plus grande dimension appartient à la gamme allant de 1 à 1000 nm, de préférence de 20 à 50 nm. Cette plus grande dimension peut être mesurée sur des clichés pris au microscope électronique à transmission ou par diffusion dynamique de la lumière.

Après dépôt, le substrat sur lequel le complexe est déposé est traité thermiquement, le plus souvent à une température appartenant à la gamme allant de 80 à 120°C pour permettre au matériau d'adhérer sur la surface du substrat par des liaisons covalentes, par exemple de type siloxane Si-O-Si ou métaloxane M-O-Si (M = Cu ou AI) selon la nature du substrat. Le plus souvent, il se produit également une polymérisation entre complexes avec formation de ponts Si-O-Si. Le traitement thermique, peut par exemple durer de 30 minutes à 4 heures, par exemple il est de l'ordre de 2 heures.

Il est possible que, pour chaque complexe greffé, un ou plusieurs des atomes de silicium situés en bout de chaine d'au moins un des groupements Z₁ soi(en)t lié(s) par trois liaisons covalentes -O-Si au substrat. Mais le plus souvent, un tel atome de silicium ne sera lié au substrat que par un ou deux ponts O-Si, du fait de la présence de groupement(s) ORₐ résiduel(s). Il est également possible que, pour chaque complexe greffé, seulement un ou plusieurs des atomes de silicium situés en bout de chaine des groupements Z₁ soi(en)t lié(s) au substrat.

Des revêtements homogènes, sous la forme de film ou de couches continues, de faibles rugosités avec des épaisseurs comprises entre quelques nanomètres et quelques micromètres peuvent ainsi être préparés.

Dans le cadre de l'invention, il est possible de réaliser un dépôt de complexes de terre rare (A) qui vont être liés de manière covalente à un substrat de type support plan ou de forme complexe, ou particules. Une telle liaison chimique covalente a l'avantage de conduire à une liaison stable et de garantir une meilleure homogénéité du dépôt et une bonne dispersion spatiale. De plus, la liaison covalente existant entre le substrat inorganique et le complexe organo-métallique (A) conduit à des objets ou matériaux manufacturés fortement émissifs et facilement manipulables.

Il est possible que des complexes différents soient greffés en surface du substrat. Il est également possible que les complexes greffés ne diffèrent que par la nature de la terre rare qui est piégée. En particulier, il est possible d'utiliser une association de plusieurs terres rares et en particulier de deux ou trois terres rares. A titre d'exemple, on pourra greffer en surface des complexes, de manière à avoir un mélange d'ions Yb³⁺/Er3⁺ ou Yb³⁺/Tm³⁺ ou Yb³⁺/Nd³⁺, avec des rapports molaires variables.

Les ions de terre rare piégés peuvent être excités par un rayonnement UV, proche UV, bleu, vert, rouge ou infrarouge, c'est-à-dire respectivement dans une gamme de longueur d'onde comprise entre 250 nm et 350 nm, entre 350 nm et 400 nm, entre 400 nm et 490 nm, entre 500 nm et 530 nm, entre 700 nm et 850 nm ou entre 900 nm et 1200 nm. Les matériaux hybrides selon l'invention ont alors pour propriétés d'émettre dans le bleu, le vert, le rouge ou l'infrarouge dans une gamme de longueur d'onde comprise entre 400 et 500 nm pour le bleu, entre 510 nm et 560 nm pour le vert, entre 600 et 630 nm pour le rouge et entre 900 et 1200 nm pour l'IR; plus précisément à environ 440 nm ou 480 nm pour le bleu, à environ 540 nm ou 550 nm pour le vert, à environ 610 nm, 620 nm ou 650 nm pour le rouge et à environ 915 nm, 980 nm, 110 nm ou 1500 nm pour l'infrarouge avec un bon rendement quantique.

Les ions ciblés sont Tm³⁺ ou le Pr³⁺ pour le bleu, Er³⁺, Tb³⁺ ou Ho³⁺ pour le vert, Er³⁺, Ho³⁺ ou Eu³⁺ pour le rouge et Yb³⁺ ou Nd³⁺ pour l'infrarouge.

Selon la longueur d'onde d'excitation sélectionnée, deux ou trois ions Ln³⁺ peuvent être combinés pour obtenir une émission de la fluorescence optimale. A titre d'exemple:
- il est avantageux d'introduire le couple Yb³⁺ et Er³⁺ dans les matériaux selon l'invention, avec un rapport molaire de 20:2 environ pour obtenir une émission verte intense à 540 nm sous excitation à 980 nm ;
- il est avantageux d'introduire le couple Yb³⁺ et Tm³⁺ dans les matériaux selon l'invention, avec un rapport molaire de 20:3 environ pour obtenir une émission bleue intense à 440 nm sous excitation à 915 nm ou 980 nm ;
- il est avantageux d'introduire les ions Gd³⁺, Yb³⁺ et Er³⁺ dans les matériaux selon l'invention, avec un rapport molaire de 20:20:2 environ pour accroître le rendement de fluorescence de l'Er³⁺ simultanément dans le vert à environ 540 nm et dans le rouge à environ 650 nm sous excitation à 980 nm.
- il est avantageux d'introduire les ions Gd³⁺, Yb³⁺ et Tm³⁺ dans les matériaux selon l'invention, avec un rapport molaire de 20:10:2 environ pour obtenir une émission UV vers 312 nm sous excitation à 980 nm.

Dans le cas où le substrat inorganique correspond à des particules de silice submicroniques, on utilisera, de préférence, des particules de silice de distributions monomodales préparées par procédé sol-gel. De manière avantageuse, l'un des deux protocoles suivants sera utilisé, en fonction du diamètre visé: la méthode de Stöber pour des diamètres supérieurs à 100 nm ou la méthode de microémulsion inverse pour des diamètres inférieurs à 100 nm.

L'invention peut donc conduire à des nanoparticules luminescentes à la taille et aux propriétés optiques contrôlées, ce qui présente notamment un intérêt pour le marquage de cellules vivantes et l'observation de leur structure et fonction. Les particules hybrides basées sur l'association covalente des complexes organiques de terres rares (A) selon l'invention avec des particules, en particuliers des nanoparticules de silice, combinent la réponse lumineuse élevée des marqueurs moléculaires et la stabilité dans le temps des caractéristiques physico-chimiques des marqueurs inorganiques (principalement "quantum dots").

Selon un mode de mise en oeuvre particulier de l'invention, qui peut être appliqué dans tous les cas, et notamment dans le cas où le substrat se trouve sous la forme de particules de silice, et plus précisément de nanoparticules, les complexes greffés en surface du substrat sont recouverts d'une couche de silice, éventuellement fonctionnalisée, par exemple par des fonctions -NH₂, -C≡CH, -COOH, -N₃ ou -OH. Cette couche de silice assure une protection des complexes de terre rare qui ne sont donc pas directement en contact avec le milieu extérieur. Une telle couche de silice peut être déposée à partir d'un précurseur du type tetraalcoxysilane ou organotrialcoxysilane quand un groupe fonctionnel doit être introduit, selon des procédés sol-gel classiques bien connus de l'homme de l'art. A titre d'exemple de précurseurs pouvant être utilisés, on peut citer le tetraméthoxysilane, le tétraéthoxysilane, l'APTES (3-aminopropyl(triéthoxyl)silane) permettant d'introduire une fonction -NH₂ en surface de la couche de silice, ou l'O-(propargyloxy)-N-(troéthoxysilylpropyl) uréthane permettant d'introduire une fonction -C≡CH en surface de la couche de silice.

La présence de fonctions du type -NH₂, -COOH, -OH, -N₃, -C≡CH, -C≡N sur la couche de silice venant recouvrir la couche de complexe (A) déposée sur le substrat est particulièrement avantageuse pour réaliser des réactions de couplage, permettant le greffage ultérieur de molécules d'intérêt tels que des ligands biologiques ou d'autres molécules d'intérêt. A titre d'exemple de ligands biologiques, on peut citer les protéines, anticorps, antigènes, nucléotides, sucres, vitamines, hormones, ou plus généralement toute molécule organique présentant un intérêt pour la vectorisation biologique. En particulier, un ligand biologique s'entend d'un composé qui possède au moins un site de reconnaissance lui permettant de réagir avec un site ou une molécule cible d'intérêt biologique. Il est ainsi possible de greffer par liaison covalente, sur la couche de silice, des molécules vectorisantes capables de cibler spécifiquement des marqueurs membranaires impliqués dans la résistance aux traitements dans les tumeurs solides.

Dans le cas où la couche de silice est fonctionnalisée en surface par des fonctions N₃ ou -C≡CH, les ligands biologiques seront greffés par click chemistry. Pour plus de détails sur les réactions de « click chemistry » qui font intervenir une réaction de cycloaddition d'un dérivé azido sur un dérivé alcyne à température ambiante, on pourra se référer à Timothy R. Chan, Robert Hilgraf, K. Barry Sharpless, and Valery V. Fokin Org. Lett, 2004, 6 (17), 2853-2855. De telles réactions peuvent être réalisées notamment avec une catalyse par le cuivre (I)

L'invention permet d'avoir des marqueurs et traceurs ayant une fluorescence intense proche de celle des fluorophores organiques et une insensibilité face à l'environnement externe qui peut être identique à celle des Q-dots.

Les matériaux selon l'invention pourront être utilisés dans différentes applications, notamment dans le domaine de l'éclairage, de l'affichage, de la traçabilité et notamment du marquage anti-contrefaçon ou en biologie ou dans le secteur solaire photovoltaïque.

L'utilisation des matériaux selon l'invention incorporant des complexes de terres rares greffés par liaison covalente sur un substrat autorise diverses approches pour l'imagerie. Les deux plus intéressantes sont :
- L'imagerie biphotonique, notamment dans la fenêtre de faible absorption et de diffusion de la lumière excitatrice par les tissus (0,9-1,2 µm). On peut parfaitement envisager des excitations dans cette fenêtre de transparence pour imager en profondeur dans l'IR ou le visible. Cette voie est actuellement privilégiée pour l'imagerie *in vivo* du petit animal. D'autre part un choix ciblé de centres émetteurs ouvre une autre perspective, celle de pouvoir coupler de la thérapie à l'imagerie en convertissant au voisinage des tumeurs malignes le rayonnement excitateur IR en rayonnement UV ou proche UV susceptible d'être fortement absorbé et de provoquer ainsi la lésion irréversible de la tumeur ciblée.
- L'imagerie Résolue dans le Temps, qui par l'utilisation d'un rayonnement excitateur à impulsions couplée à des chromophores possédant des états électroniques excités de durées de vie longues permettrait de séquencer l'imagerie dans le temps.

Les exemples ci-dessous qui n'ont aucun caractère limitatif permettent de mieux comprendre l'invention et donnent une illustration des synthèses qui pourront être mises en oeuvre pour leur préparation. L'homme du métier sera à même d'y apporter les modifications adéquates, en fonction des complexes et matériaux qu'il souhaite réaliser.

### 1- Synthèse des ligands organiques

### 1a - Schéma de synthèse

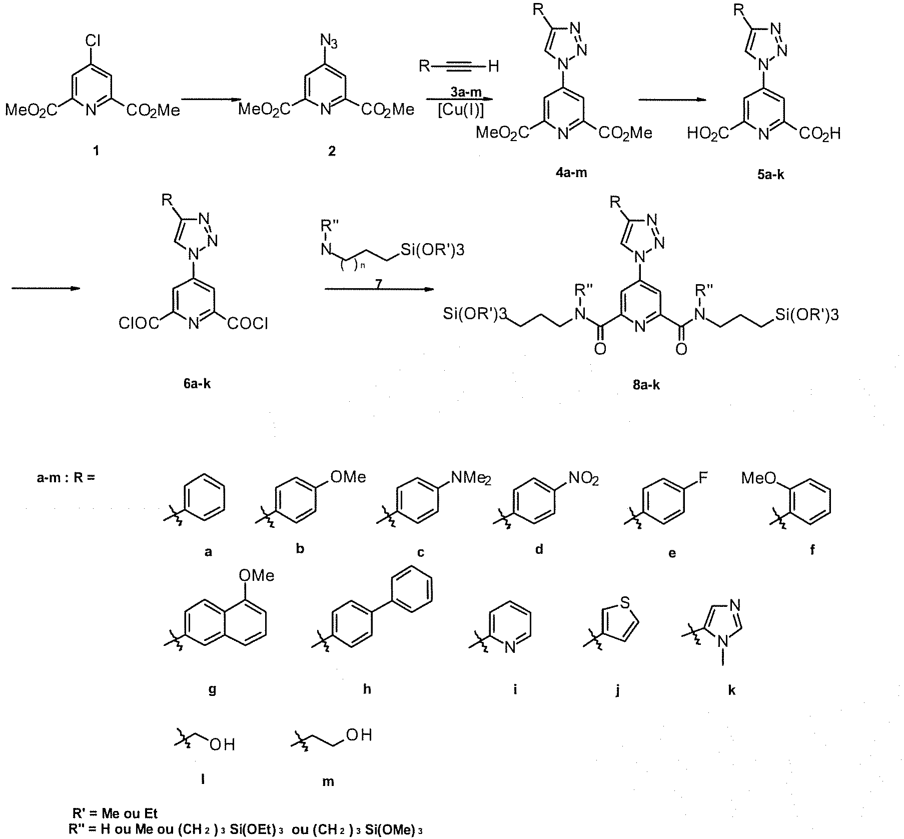

Les composés **8l** et **8m** sont préparés selon le schéma de synthèse ci-dessus, mais au moyen de deux étapes supplémentaires : une protection de la fonction alcool au niveau de l'intermédiaire **4** et, in fine, la déprotection de cette fonction.

### 1b - Modes Opératoires

### Synthèse du 4-azidopyridine-2,6-dicarboxylate de diméthyle 2.

A une suspension de 2,2 g (9,56 mmol) de 4-chloropyridine-2,6-dicarboxylate de diméthyle **1** (préparé selon Tetrahedron 2005, 61, 1755) dans 30 mL de DMF, on ajoute 6,2 g (10éq.) d'azoture de sodium. La suspension résultante est chauffée à 50°C pendant 24 heures puis refroidie à température ambiante, avant d'être versée dans 80 mL d'eau sous agitation vigoureuse. Le composé 2 précipite immédiatement et est isolé sous forme d'un solide blanc (2.0 g, 90%) par filtration sur verre fritté.
¹H NMR (400 MHz, DMSO-D₆) δ 7.87 (s, 2H), 3.92 (s, 6H)

### Procédures générales de synthèse des triazoles 4 par réaction de CuAAC.

### - Méthode A : utilisation de (Cu(I)/THPTA) comme catalyseur.

A une suspension de 4-azidopyridine-2,6-dicarboxylate de diméthyle **2** (236 mg, 1 mmol) dans 10 mL de méthanol, on ajoute 1,1 mmol d'alcyne commercial **3.** On additionne ensuite 200 microL d'une solution 0,1M de complexe (Cu(I)/THPTA, 2 mol%) - préparé selon Eur. J. Inorg. Chem. 2008, 298 et Angew Chem. Int. Ed. 2009, 48, 9879 - puis 600 microL d'une solution aqueuse 0,1 M d'acide ascorbique (6 mol%). La couleur du mélange réactionnel vire du vert au jaune. Lorsque la couleur du mélange redevient verte, l'avancement de la réaction est vérifié par chromatographie sur couche mince (disparition de l'azoture **2,** éluant : acétate d'éthyle/cyclohexane 1v/1v). Si la réaction est incomplète, on procède à un nouvel ajout de solution aqueuse d'acide ascorbique (0,1 M, 600 microL, 6 mol%). Les triazoles **4** précipitent au fur et à mesure de lors formation et sont isolés purs après filtration sur verre fritté et lavage au méthanol froid puis à l'éther de diéthyle (solides blancs à jaunes selon la nature de l'alcyne **3** employé, rendements : 75% à 100%). - Méthode B : utilisation de [CuCl(SIMes)(4,7-dichloro-1,10-phénanthroline)] comme catalyseur.

A une suspension de 4-azidopyridine-2,6-dicarboxylate de diméthyle **2** (236 mg, 1 mmol) dans 10 mL de méthanol, on ajoute 1,1 mmol d'alcyne commercial **3.** On additionne ensuite 13 mg (2 mol%) de complexe [CuCl(SIMes)(4,7-dichloro-1,10-phenanthroline)] (préparé selon Eur. J. Org. Chem. 2010, 3507). Les triazoles **4** précipitent au fur et à mesure de leur formation. Lorsque la réaction est terminée (disparition totale de l'azoture **2** vérifiée par chromatographie sur couche mince), les triazoles **4** sont isolés purs après filtration sur verre fritté et lavage au méthanol froid puis à l'éther de diéthyle (solides blancs à jaunes selon la nature de l'alcyne **3** employé, rendements : 84% à 100%).

Diméthyl 4-(4-phényl-1*H-*,2,3-triazol-1-yl)pyridine-2,6-dicarboxylate **4a :**
¹H RMN (400 MHz, CDCl₃) δ 8.81 (s, 2H), 8,46 (s, 1H), 7.97 (d, J = 8 Hz, 2H), 7.53(t, J = 8 Hz, 2H), 7.47 (d, J = 8 Hz, 1H), 4.12 (s, 6H).

Diméthyl 4-[4-(4-méthoxyphényl)-1*H*-1,2,3-triazol-1-yl]pyridine-2,6-dicarboxylate **4b :**
¹H RMN (400 MHz, DMSO-D₆) δ : 9.73 (1H, s), 8.80 (2H, s), 7.91 (2H, d, J = 9 Hz), 7.10 (2H, d, J = 9 Hz), 3.99 (6H, s), 3.82 (3H, s).

Diméthyl 4-[4-(4-diméthylaminophényl)-1*H*-1,2,3-triazol-1-yl]pyridine-2,6-dicarboxylate **4c :**
¹H RMN (400 MHz, DMSO-D₆) δ 9.60 (s, 1H), 8.79 (s, 2H), 7.79 (d, J = 9 Hz, 2H), 6.84 (d, J = 9 Hz), 4.00 (s, 6H), 2.98 (s, 6H).

Diméthyl 4-[4-(4-nitrophényl)-1*H*-,2,3-triazol-1-yl]pyridine-2,6-dicarboxylate **4d :**
¹H RMN (400 MHz, DMSO-D₆) δ 10.1 (1H, s), 8.83 (2H, s), 8.42 (2H, d, J = 9 Hz), 25 (2H, d, J = 9 Hz), 4.00 (6H, s).

Diméthyl 4-[4-(4-fluorophényl)-1*H*-,2,3-triazol-1-yl]pyridine-2,6-dicarboxylate **4e :**
¹H RMN (400 MHz, DMSO-D₆) δ 9.83 (s, 1H), 8.80 (s, 2H), 8.02 (dd, J = 9 Hz, J = 6 Hz, 2H), 7.39 (t, J = 8.8 Hz, 2H), 4.00 (s, 6H).

Diméthyl 4-[4-(2-méthoxyphényl)-1*H*-1,2,3-triazol-1-yl]pyridine-2,6-dicarboxylate **4f :**
¹H RMN (400 MHz, DMSO-D₆) δ 9.43 (s, 1H), 8.88 (s, 2H), 8.21 (dd, J = 8 Hz, J =1.5 Hz, 1H), 7.42 (dt, J = 8 Hz, J = 1.5 Hz, 1H), 7.20 (d, J = 8 Hz, 1H), 7.11 (t, J = 8 Hz, 1H), 4.00 (s, 3H), 3.99 (s, 6H).

Diméthyl 4-[4-(6-méthoxy-2-naphtyl)-1*H*-1,2,3-triazol-1-yl]pyridine-2,6-dicarboxylate **4g :**
¹H RMN (400 MHz, DMSO-D₆) δ 9.87 (s, 1H), 8.81 (s, 2H), 8.45 (s, 1H), 8.03 (dd, J = 8.5 Hz, J =1.5 Hz, 1H), 7.95 (d, J = 8.5 Hz, 1H), 7.91 (d, J = 9.5 Hz, 1H), 7.36 (d, J=2.5 Hz, 1H), 7.22 (dd, J = 9.5 Hz, J = 2.5 Hz, 1H), 4.00 (s, 6H), 3.90 (s, 3H).

Diméthyl 4-[4-(4-biphényl)-1*H*-,2,3-triazol-1-yl]pyridine-2,6-dicarboxylate **4h :**
¹H RMN (400 MHz, DMSO-D₆) δ 9.90 (s, 1H), 8.83 (s, 2H), 8.08 (d, J = 8.5 Hz, 2H), 7.87 (d, J = 8,5 Hz, 2H), 7.76 (d, J = 7.5 Hz, 2H), 7.50 (t, J = 7 Hz, 2H), 7.40 (t, J = 7 Hz, 1H), 4.00 (s, 6H).

Diméthyl 4-[4-(2-pyridinyl)-1*H*-1,2,3-triazol-1-yl]pyridine-2,6-dicarboxylate **4i** :
¹H RMN (400 MHz, DMSO-D₆) δ 9.88 (s, 1H), 8.91 (s, 2H), 8.69 (ddd, J = 5 Hz, J = 2 Hz, J = 1 Hz, 1H), 8.15 (dt, J =7.5 Hz, J = 1 Hz, 1H), 7.98 (dt, J =7.5 Hz, J = 2 Hz, 1H), 7.44 (ddd, J = 7.5 Hz, J = 5 Hz, J = 1 Hz, 1H), 3.99 (s, 6H).

Diméthyl 4-[4-(3-thiophényl)-1*H*-1,2,3-triazol-1-yl]pyridine-2,6-dicarboxylate **4j :**
¹H RMN (400 MHz, DMSO-D₆) δ 9.68 (s, 1H), 8.77 (s, 2H), 8.02 (dd, J = 7 Hz, J = 1 Hz, 1H), 7.74 (dd, J = 5 Hz, J₂ = 3 Hz, 1H), 7.61 (dd, J = 5 Hz, J = 1 Hz, 1H), 3.99 (s, 6H).

Diméthyl 4-[4-(5(1-méthyl)-1*H*-imidazolyl)-1*H*-1,2,3-triazol-1-yl]pyridine-2,6-dicarboxylate **4k :**
¹H RMN (400 MHz, DMSO-D₆) δ 9.59 (s, 1H), 8.85 (s, 2H), 7.80 (s, 1H), 7.39 (s, 1H), 3.99 (s, 6H), 3.90 (s, 3H).

Diméthyl 4-(4-hydroxyméthyl-1*H*-1,2,3-triazol-1-yl)pyridine-2,6-dicarboxylate **4l :**
¹H RMN (400 MHz, CDCl₃) δ 8.73 (s, 2H), 8.26 (s, 1H), 4.96 (s, 2H), 4.08 (s, 6H).

Diméthyl 4-[4-(2-hydroxyéthyl)-1*H*-1,2,3-triazol-1-yl]pyridine-2,6-dicarboxylate **4m :**
¹H RMN (400 MHz, DMSO-D₆) δ 9.11 (s, 1H), 8.75 (s, 2H), 4.79 (t, J = 5.5 Hz, 1H), 3.98 (s, 6H), 3.73 (q, J = 6.5 Hz, 2H), 2.89 (t, J = 6.5 Hz, 2H).

### Procédure générale de synthèse des diacides 5 par saponification des diesters 4.

A une suspension de 1 mmol de diester **4** dans 5 mL d'eau sont additionnés 4 mL d'une solution aqueuse d'hydroxyde de sodium 1M (4 mmol). La solution résultante est chauffée à 60°C pendant 4h puis refroidie à température ambiante avant acidification (pH < 2) réalisée par ajout goutte à goutte d'une solution d'acide chlorhydrique 3M. Les diacides **5** ainsi formés précipitent et sont isolés purs après filtration sur verre fritté suivie d'un lavage à l'eau froide puis d'un séchage sous pression réduite d'une pompe à palettes (solides blancs à jaunes selon la nature du diester **4** employé, rendements : 95% à 100%).

Acide 4-(4-phényl-1*H*-1,2,3-triazol-1-yl)pyridine-2,6-dicarboxylique **5a :**
¹H RMN (400 MHz, D₂O/NaOD) δ 7.65 (s, 2H), 7.03 (s, 2H), 6.77-6.75 (m, 3H).

Acide 4-[4-(4-méthoxyphényl)-1*H*-1,2,3-triazol-1-yl]pyridine-2,6-dicarboxylique **5b :**
¹H RMN (400 MHz, D₂O/NaOD) δ 7.56 (s, 2H), 6.81 (s, 2H), 6.03 (s, 2H), 3.20 (s, 3H).

Acide 4-[4-(4-diméthylaminophényl)-1*H*-1,2,3-triazol-1-yl]pyridine-2,6-dicarboxylique **5c :**
¹H RMN (400 MHz, D₂O/NaOD) δ 7.72 (s, 2H), 6.88 (br s, 2H), 5.92 (br s, 2H), 2.29 (s, 6H).

Acide 4-[4-(4-nitrophényl)-1*H*-1,2,3-triazol-1-yl]pyridine-2,6-dicarboxylique **5d :**
¹H RMN (400 MHz, DMSO-D₆) δ 10.09 (1H, s), 8.28 (2H, s), 8.42 (2H, d, J = 9 Hz), 8.25 (2H, d, J = 9 Hz).

Acide 4-[4-(4-fluorophényl)-1*H*-1,2,3-triazol-1-yl]pyridine-2,6-dicarboxylique **5e :**
¹H RMN (400 MHz, D₂O/NaOD) δ 8.47 (s, 1H), 7.99 (s, 2H), 7.36 (br s, 2H), 6.78 (t, J = 8 Hz, 2H).

Acide 4-[4-(2-méthoxyphényl)-1*H*-1,2,3-triazol-1-yl]pyridine-2,6-dicarboxylique **5f :**
¹H RMN (400 MHz, D₂O/NaOD) δ 7.86 (s, 1H), 7.66 (s, 2H), 7.37 (d, J = 7 Hz,1H), 6.69 (t, J =6.5 Hz, 1H), 6.44 (t, J = 6.5 Hz, 1H), 6.25 (d, J=6 Hz, 1H), 3.56 (s, 3H).

Acide 4-[4-(6-méthoxy-2-naphtyl)-1H-1,2,3-triazol-1-yl]pyridine-2,6-dicarboxylique **5g :**
¹H RMN (400 MHz, D₂O/NaOD) δ 7.86 (s, 1H), 7.66 (s, 2H), 7.37 (d, J = 7 Hz,1H), 6.69 (t, J = 6.5 Hz, 1H), 6.44 (t, J = 6.5 Hz, 1H), 6.25 (d, J = 6 Hz, 1H), 3.56 (s, 3H).

Acide 4-[4-(4-biphényl)-1*H*-1,2,3-triazol-1-yl]pyridine-2,6- dicarboxylique **5h** :
¹H RMN (400 MHz, D₂O/NaOD) δ 7.93 (s, 2H), 7.53 (d, J = 8.5 Hz, 2H), 7.41 (d, J=8.5 Hz, 2H), 7.23 (d, J = 7 Hz, 2H), 7.03 (m, 3H).

Acide 4-[4-(2-pyridinyl)-1*H*-1,2,3-triazol-1-yl]pyridine-2,6- dicarboxylique **5i :** ¹H RMN (400 MHz, D₂O/NaOD) δ 8.37 (s, 1H), 8.01 (s, 1H), 7.87 (s, 2H), 7.40 (q, J= 5.5 Hz, 1H), 7.34 (q, J = 5.5 Hz, 1H), 6,95 (br s, 1H)

Acide 4-[4-(3-thiophényl)-1*H*-1,2,3-triazol-1-yl]pyridine-2,6-dicarboxylique **5j :**
¹H RMN (400 MHz, D₂O/NaOD) δ 7.80 (s, 2H), 7.13 (s, 1H), 6.94 (s, 1H), 6.76 (s, 1H).

Acide 4-[4-(5(1-méthyl)-1*H*-imidazoiy))-1*H*-1,2,3-triazol-1-yl]pyridine-2,6-dicarboxylique **5k :**
¹H RMN (400 MHz, D₂O/NaOD) δ 8.11 (s, 1H), 7.99 (s, 2H), 7.51 (m, 2H), 7.04 (td, J = 5.5 Hz, J = 2 Hz, 1H).

Acide 4-(4-hydroxyméthyl-1*H*-1,2,3-triazol-1-yl)pyridine-2,6-dicarboxylique **5l :**
¹H RMN (400 MHz, D₂O) δ 9.20 (1H, s), 8.73 (2H, s), 4.63 (2H, s).

4-[4-(2-hydroxyéthyl)-1*H*-1,2,3-triazol-1-yl]pyridine-2,6- dicarboxylic acid **5m** :
¹H RMN (400 MHz, D₂O/NaOD) δ 8.40 (s, 1H), 8.27 (s, 2H), 3.86 (br s, 2H), 2.95 (t, J = 6.4 Hz, 2H).

### Procédure générale de synthèse des diamides 8 à partir des diacides 5.

La séquence réactionnelle est réalisée sous atmosphère d'argon. A une suspension de 2 mmol de diacide **5** dans 30 mL de chlorure de thionyle fraîchement distillé sont additionnés 150 microL de *N,N*-diméthylformamide. La suspension résultante est chauffée à reflux jusqu'à dissolution complète du diacide puis pendant 4 heures supplémentaires. Après refroidissement à température ambiante, le chlorure de thionyle est totalement éliminé par évaporation sous pression réduite (pompe à palettes) pour fournir l'intermédiaire di-chlorure de diacide **6.** Celui-ci est solubilisé dans 50 mL d'éther rigoureusement anhydre et la solution est refroidie à 0°C avant addition de 8 mmol de pyridine anhydre puis de 4 mmol d'amine **7.** Le mélange résultant est agité pendant 15 minutes à 0°C puis pendant 3 heures à température ambiante. Après filtration de la suspension ainsi obtenue (élimination du chlorhydrate de pyridinium formé) suivie de l'élimination des solvants sous pression réduite (co-évaporation en présence de CCl₄ anhydre à l'évaporateur rotatif, puis séchage à la pompe à palettes), on obtient les diamides correspondants **8.**

*N,N,N'*,*N'*-tetrakis[(3-triéthylsilyloxy)propyl]-4-(4-phényl-1*H*-1,2,3-triazol-1-yl)pyridine-2,6-dicarboxamide **8a :**
¹H RMN (400 MHz, DMSO-D₆) 9.85 (s, 2H), 8,81 (s, 1H), 7.97 (d, J = 8 Hz, 2H), 7.51 (t, J = 8 Hz, 2H), 7.48 (d, J = 8 Hz, 1H), 3.89 (q, J = 7 Hz, 12H), 3.68 (q, J = 7 Hz, 12H), 3.39 (m, 4H), 3.24 (m, 4H), 1.76 (m, 4H), 1.64 (m, 4H), 1.25 (t, J = 7 Hz, 18H), 1.21 (t, J = 6.5 Hz, 18H), 0.65 (m, 4H), 0.44 (m, 4H).

*N,N,N',N*'-tetrakis[(3-triéthylsilyloxy)propyl]-4-[4-(4-méthoxyphényl)-1*H-*1,2,3-triazol-1-yl]pyridine-2,6-dicarboxamide **8b :**
¹H RMN (400 MHz, DMSO-D₆) 9.70 (s, 1H), 8.77 (s, 2H), 7.92 (d, J = 9 Hz, 2H), 7.09 (d, J = 9 Hz, 2H), 3.89 (q, J = 6.5 Hz, 12H), 3.83 (s, 3H), 3.67 (q, J = 6.5 Hz, 12H), 3.39 (m, 4H), 3.30 (m, 4H), 1.75 (m, 4H), 1.68 (m, 4H), 1.23 (t, J = 6.5 Hz, 18H), 1.21 (t, J = 6.5 Hz, 18H), 0.59 (m, 4H), 0.43 (m, 4H).

*N,N,N',N'*-tetrakis[(3-triéthylsilyloxy)propyl]-4-[4-(4-diméthylaminophényl)-1*H*-1,2,3-triazol-l-yl]pyridine-2,6- dicarboxamide **8c :**
¹H RMN (400 MHz, DMSO-D₆) 9.61 (s, 1H), 8.78 (s, 2H), 7.78 (d, J = 9 Hz, 2H), 6.84 (d, J = 9 Hz, 2H), 3.87 (q, J = 7 Hz, 12H), 3.65 (q, J = 7 Hz, 12H), 3.29 (m, 8H), 2.98 (s, 6H), 1.73 (m, 4H), 1.68 (m, 4H), 1.22 (br t, J = 7 Hz, 36H), 0.64 (m, 4H), 0.53 (m, 4H).

*N,N,N',N'*-tetrakis[(3-triéthylsilyloxy)propyl]-4-[4-(4-nitrophényl)-1*H*-1,2,3-triazol-1-yl]pyridine-2,6- dicarboxamide **8d** :
¹H RMN (400 MHz, DMSO-D₆) δ 10.09 (s, 1H), 8.85 (s, 2H), 8.41 (d, J = 9 Hz, 2H), 8.25 (d, J = 9 Hz, 2H), 3.90 (q, J = 7 Hz, 12H), 3.64 (q, J = 7 Hz, 12H), 3.30 (m, 8H), 1.76 (m, 4H), 1.67 (m, 4H), 1.26 (t, J = 7 Hz, 18H), 1.21 (t, J = 7 Hz, 18H), 0.66 (m, 4H), 0.52 (m, 4H).

*N,N,N',N*'-tetrakis[(3-triéthylsilyloxy)propyl]-4-[4-(4-fluorophényl)-1*H*-1,2,3-triazol-1-yl]pyridine-2,6- dicarboxamide 8e :
¹H RMN (400 MHz, DMSO-D₆) δ 9.82 (s, 1H), 8.77 (s, 2H), 7.98 (dd, J = 9 Hz, J = 6 Hz, 2H), 7.39 (t, J = 9 Hz, 2H), 3.88 (q, J = 7 Hz, 12H), 3.78 (q, J = 7 Hz, 12H), 3.40 (m, 4H), 3.31 (m, 4H), 1.75 (m, 4H), 1.67 (m, 4H), 1.26 (t, J = 7 Hz, 18H), 1.19 (t, J = 7 Hz, 18H), 0.65 (m, 4H), 0.51 (m, 4H).

*N,N,N',N'*-tetrakis[(3-triéthylsilyloxy)propyl]-4-[4-(2-méthoxyphényl)-1*H-*1,2,3-triazol-1-yl]pyridine-2,6- dicarboxamide **8f** :
¹H RMN (400 MHz, DMSO-D₆) δ ·9.40 (s, 1H), 8.86 (s, 2H), 8.21 (dd, J = 8 Hz, J = 2 Hz, 1H), 7.43 (dt, J = 8 Hz, J = 2 Hz, 1H), 7.18 (d, J = 8 Hz, 1H), 7.10 (t, J = 8 Hz, 1H), 3.98 (s, 3H), 3.76 (br q, J = 7 Hz, 24H), 3.35 (m, 4H), 3.26 (m, 4H), 1.70 (m, 8H), 1.23 (br t, J = 7 Hz, 36H), 0.63 (m, 4H), 0.46 (m, 4H).

*N,N,N',N'*-tetrakis[(3-triéthylsilyloxy)propyl]-4-[4-(6-méthoxy-2-naphtyl)-1*H-*1,2,3-triazol-1-yl]pyridine-2,6- dicarboxamide **8g** :
¹H RMN (400 MHz, DMSO-D₆) δ 9.87 (s, 1H), 8.78 (s, 2H), 8.44 (s, 1H), 8.04 (dd, J = 9 Hz, J = 2 Hz, 1H), 7.95 (d, J = 9 Hz, 1H), 7.90 (d, J = 9 Hz, 1H), 7.36 (d, J = 2 Hz, 1H), 7.20 (dd, J = 9 Hz, J = 2 Hz, 1H), 3.89 (s, 3H), 3.82 (q, J = 7 Hz, 12H), 3.70 (q, J = 7 Hz, 12H), 3.32 (br m, 8H), 1.75 (m, 4H), 1.68 (m, 4H), 1.23 (t, J = 7 Hz, 18H), 1.18 (t, J = 6.5 Hz, 18H), 0.63 (m, 4H), 0.45 (m, 4H).

*N,N,N',N'*-tetrakis[(3-triéthylsilyloxy)propyl]-4-[4-(4-biphényl)-1*H*-1,2,3-triazol-1-yl]pyridine-2,6- dicarboxamide **8h** :
¹H RMN (400 MHz, DMSO-D₆) δ 9.90 (s, 1H), 8.79 (s, 2H), 8.09 (d, J = 8.5 Hz, 2H), 7.87 (d, J = 8,5 Hz, 2H), 7.72 (d, J = 7Hz, 2H), 7.51 (t, J = 7 Hz, 2H), 7.40 (t, J = 7 Hz, 1H), 3.84 (q, J = 7 Hz, 12H), 3.67 (q, J = 7 Hz, 12H), 3.42 (m, 4H), 3.26 (m, 4H), 1.75 (m, 4H), 1.64 (m, 4H), 1.24 (t, J = 7 Hz, 18H), 1.20 (t, J = 6.5 Hz, 18H), 0.57 (br m, 8H).

*N,N,N',N'*-tetrakis[(3-triéthylsilyloxy)propyl]-4-[4-(2-pyridinyl)-1*H*-1,2,3-triazol-1-yl]pyridine-2,6- dicarboxamide **8i :**
¹H RMN (400 MHz, DMSO-D₆) δ 9.86 (s, 1H), 8.89 (s, 2H), 8.70 (dd, J = 5.5 Hz, J = 2 Hz, 1H), 8.16 (br d, J =7.5 Hz, 1H), 7.97 (dt, J =7.5 Hz, J = 2 Hz, 1H), 7.44 (dd, J = 7.5 Hz, J = 5.5 Hz, 1H), 3.90 (q, J = 7 Hz, 12H), 3.63 (q, J = 7 Hz, 12H), 3.39 (m, 4H), 3.22 (m, 4H), 1.78 (m, 4H), 1.66 (m, 4H), 1.23 (br t, J = 7 Hz, 36H), 0.63 (m, 4H), 0.45 (m, 4H).

*N,N,N',N'*-tetrakis[(3-triéthylsilyloxy)propyl]-4-[4-(3-thiophényl)-1*H*-1,2,3-triazol-1-yl]pyridine-2,6- dicarboxamide **8j :**
¹H RMN (400 MHz, DMSO-D₆) δ 9.66 (s, 1H), 8.73 (s, 2H), 8.01 (dd, J = 5 Hz, J = 2 Hz, 1H), 7.71 (dd, J = 3.5 Hz, J = 2 Hz, 1H), 7.62 (dd, J = 5 Hz, J = 3.5 Hz, 1H), 3.84 (q, J = 7 Hz, 12H), 3.73 (q, J = 7 Hz, 12H), 3.36 (m, 4H), 3.27 (m, 4H), 1.75 (m, 4H), 1.67 (m, 4H), 1.25 (t, J = 7 Hz, 18H), 1.20 (t, J = 6.5 Hz, 18H), 0.63 (m, 4H), 0.46 (m, 4H).

*N,N,N',N'*-tetrakis[(3-triéthylsilyloxy)propyl]-4-[4-(5(1-méthyl)-1*H*-imidazolyl)-1*H*-1,2,3-triazol-1-yl]pyridine-2,6- dicarboxamide **8k** :
¹H RMN (400 MHz, DMSO-D₆) δ 9.60 (s, 1H), 8.81 (s, 2H), 7.79 (s, 1H), 7.40 (s, 1H), 3.87 (s, 3H), 3.84 (q, J = 7 Hz, 12H), 3.73 (q, J = 7 Hz, 12H), 3.39 (m, 4H), 3.24 (m, 4H), 1.73 (m, 4H), 1.65 (m, 4H), 1.23 (br t, J = 7 Hz, 36H), 0.61 (m, 4H), 0.47 (m, 4H).

### 2 - Préparations des complexes de terres rares

Dans un bécher, on met le monomère fonctionnalisé (3 équivalents) en solution dans une quantité minimale de méthanol ou d'éthanol absolu (selon la nature de R') afin d'obtenir une solution limpide (environ 60 éq.).

Cette solution est laissée sous parafilm et agitation pendant 15 minutes. Dans le même temps, on pèse le nitrate ou le chlorure de lanthanide (1 équivalent) et on l'introduit dans la solution de monomère. Les différents lanthanides utilisés sont les suivants: Y³⁺, Pr³⁺ Nd³⁺, EU³⁺, Gd³⁺, Tb³⁺, Ho³⁺, Er³⁺, Tm³⁺ et Yb³⁺. Ils peuvent être introduits seuls ou sous forme de combinaison de plusieurs ions terres rares.

Cette solution est laissée sous agitation pendant 1 heure, puis on rajoute 6 équivalents d'eau distillée acidifiée avec une solution d'acide chlorhydrique de pH proche de 2.

Après avoir été homogénéisé pendant 30 minutes, le complexe hybride est conservé dans une bouteille fermée, prêt à être déposé.

Des complexes de formule (A1) ci-dessous sont ainsi obtenus :

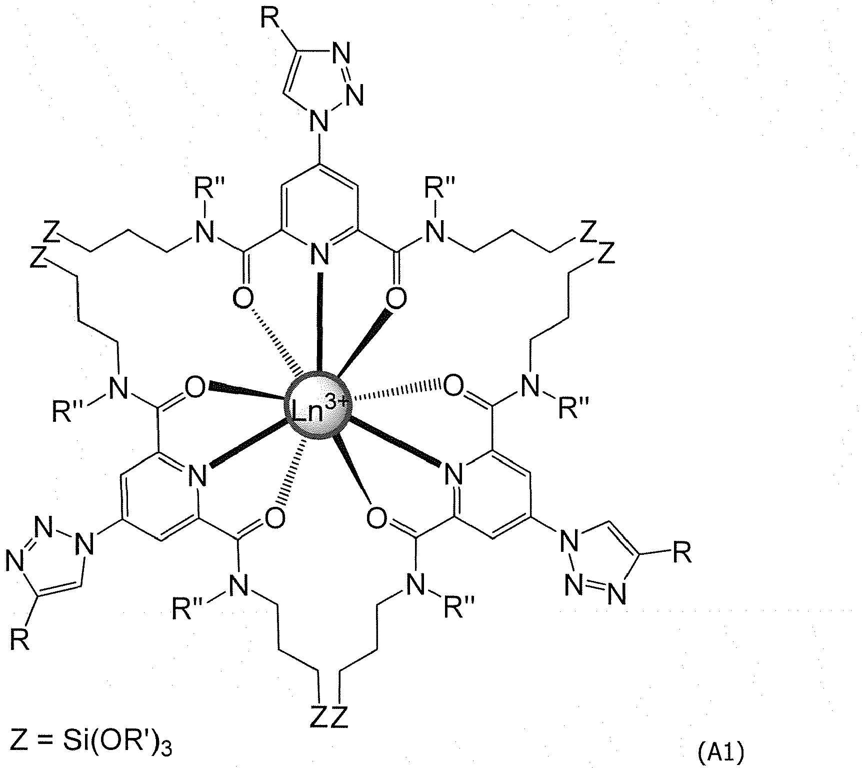

### 3 - Les revêtements luminescents

La **Figure 1** présente les images (a) MEB et (b) AFM du composé **8b** dopé Eu³⁺ déposé sur verre.
La **Figure 2** présente la mesure d'épaisseur par profilométrie d'un film du composé **8b** dopé Eu³⁺ et déposé sur verre.
La **Figure 3** présente des images MET de NPs de silice recouvertes par le composé **8b** (a) dopé Eu³⁺ et (b) dopé Tb³⁺.
La **Figure 4** présente le spectre d'émission du composé **8b** dopé Eu³⁺ déposé sur verre.
La **Figure 5** présente le spectre d'émission du composé **8b** dopé Tb³⁺ déposé sur nanoparticules de silice.

### 3a - Elaboration des revêtements

Le greffage de complexe (A) selon l'invention a pu être réalisé par trempage-retrait sur lames de verre ou sur textile en fibre de verre ou sur nanoparticules de silice. Pour le dépôt sur lame de verre ou sur textile le trempage et le retrait dans le flacon contenant l'organosilane à déposer se font à une vitesse comprise entre 1 et 30 cm.min⁻¹. Ces dépôts peuvent également être réalisés par pulvérisation avec des appareils de pulvérisations classiques. Pour recouvrir les particules de silice avec le matériau hybride, elles sont immergées pendant une durée comprise entre 1 et 24 heures dans la solution à déposer.

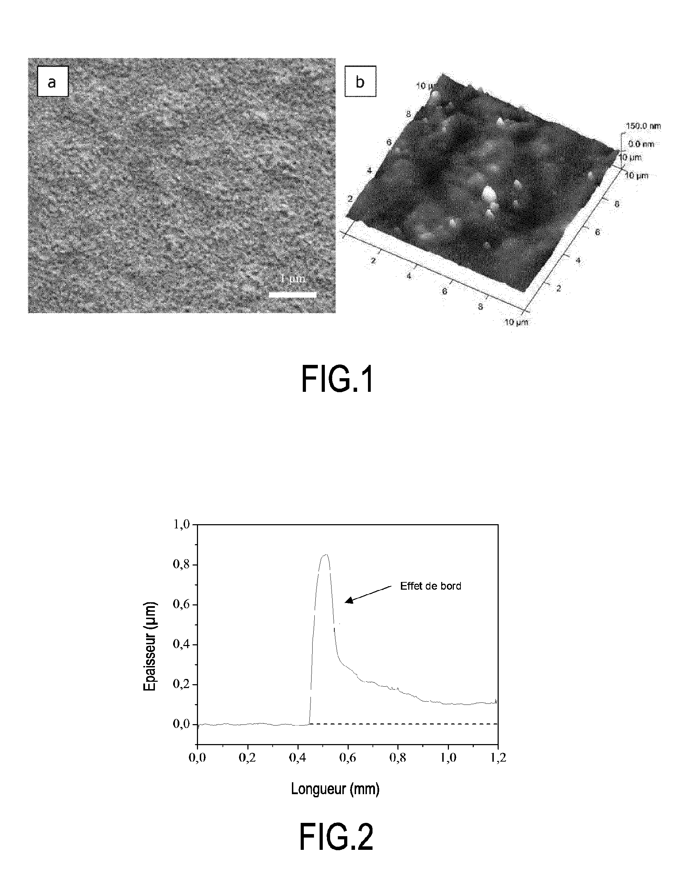

La **Figure 1** présente les images (a) MEB et (b) AFM du composé **8b** dopé Eu³⁺ déposé sur verre. Ce revêtement a été préparé à partir d'une solution de concentration 150 g.L⁻¹ en utilisant la technique de dépôt par trempage retrait avec une vitesse constante de 10 cm.min⁻¹. Le film a ensuite été traité à 110°C pendant 30 minutes.

La **Figure 2** présente la mesure d'épaisseur par profilométrie d'un film du composé **8b** dopé Eu³⁺ et déposé sur verre.

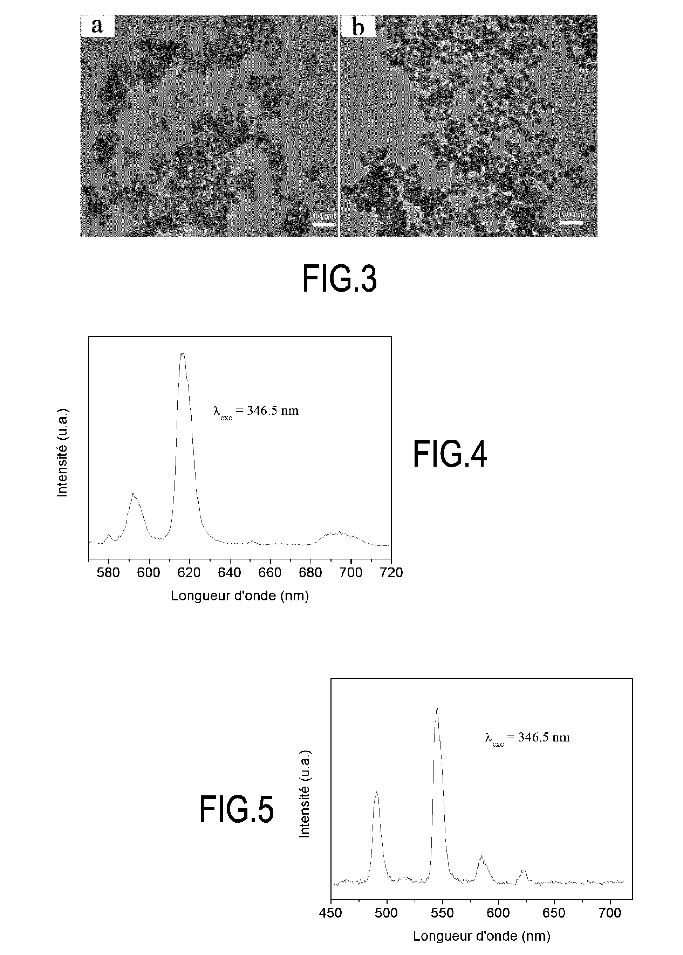

La **Figure 3** présente des images MET de NPs de silice recouvertes par le composé **8b** (a) dopé Eu³⁺ et (b) dopé Tb³⁺. Les NPs de silice ont été immergées pendant 10 heures dans la solution à déposer.

### 3b - Caractérisations optiques

Différents essais ont été réalisés permettant de mettre en évidence une luminescence verte ou rouge sous excitation UV avec différents complexes.

Le spectre d'émission du composé **8b** dopé Eu³⁺ déposé sur verre est présenté **Figure 4****.**

Le spectre d'émission du composé **8b** dopé Tb³⁺ déposé sur nanoparticules de silice est présenté **Figure 5****.**

Il apparait également que les complexes précédemment préparés dans lesquels :
1) R représente l'un des groupements suivants : 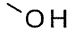 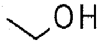 présentent :
   - un bon rendement de fluorescence,
   - une bande d'excitation fine (jusqu'à 310 nm), et
   - des possibilités d'excitation IR à 2 ou 3 photons.
2) R représente l'un des groupements suivants : 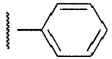 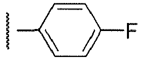 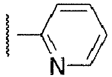 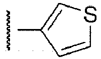 présentent :
   - un bon rendement de fluorescence,
   - une bande d'excitation large (jusqu'à 350 nm), et
   - des possibilités d'excitation IR à 2 ou 3 photons.
3) R représente l'un des groupements suivants : 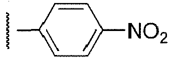 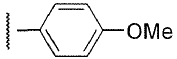 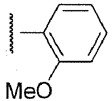 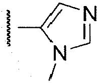 présentent :
   - un bon rendement de fluorescence,
   - une bande d'excitation large (au-delà de 400 nm), et
   - des possibilités d'excitation IR à 2 ou 3 photons.

## Revendications

1. - Complexe de formule (A) : 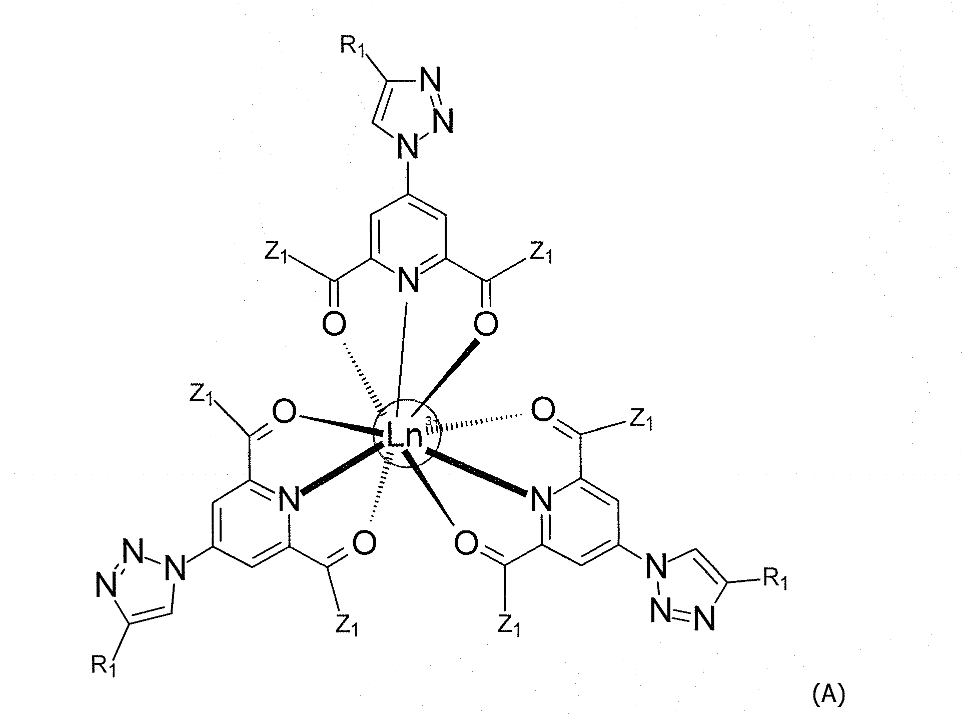 dans laquelle :
- Z₁ représente -X-L-Si(ORₐ)₃ avec X qui peut être O ou NR₄ avec R₄ qui représente un atome d'hydrogène, un groupe alkyle comprenant de 1 à 4 atomes de carbone ou un groupe -(CH₂)ₓ-Si(OR'ₐ)₃ avec x qui est égal à 1, 2, 3, 4, 5 ou 6 et R'ₐ qui représente un groupe alkyle comprenant de 1 à 4 atomes de carbone; L qui représente une chaine hydrocarbonée, saturée, substituée ou non substituée, comprenant de 1 à 6 atomes de carbone; et Rₐ qui représente un groupe alkyle comprenant de 1 à 4 atomes de carbone ;
- R₁ représente un atome d'hydrogène ou un groupe alkyle de 1 à 6 atomes de carbone pouvant comporter un ou plusieurs hétéroatomes, un groupe cycloalkyle de 3 à 7 atomes de carbone pouvant comporter un ou plusieurs hétéroatomes, un groupe aryle ou hétéroaryle, lesdits groupes alkyle, cycloalkyle, aryle et hétéroaryle pouvant être non substitués ou substitués,
- Ln est une terre rare complexée à un degré d'oxydation 3.

2. **-** Complexe selon la revendication 1 **caractérisé en ce que** Z₁ représente -X-L-Si(ORₐ)₃ avec X tel que défini à la revendication 1, L qui représente - (CH₂)_{y}- avec y qui est égale à 1, 2, 3, 4, 5 ou 6, et Rₐ qui représente un groupe alkyle comprenant de 1 à 4 atomes de carbone.

3. **-** Complexe selon la revendication 1 ou 2 **caractérisé en ce que** X représente NR₄, avec R₄ qui représente un atome d'hydrogène, un groupe méthyle, éthyle ou un groupe -(CH₂)ₓ-Si(OR'ₐ)₃ avec R'ₐ qui représente un groupe méthyle ou éthyle et x tel que défini à la revendication 1.

4. **-** Complexe selon l'une des revendications précédentes **caractérisé en ce que** R₁ représente un atome d'hydrogène ou un groupe alkyle de 1 à 6 atomes de carbone, un groupe alkyle de 1 à 6 atomes de carbone interrompu par un groupe amino, un groupe cycloalkyle de 3 à 7 atomes de carbone, un groupe phényle, naphtyle, pyridyle, thiophényle ou imidazolyle, lesdits groupes alkyle, cycloalkyle, phényle, naphtyle, pyridyle, thiophényle et imidazolyle pouvant être non substitués ou substitués par un ou plusieurs substituants choisis parmi :
- les atomes de brome, chlore, iode ou fluor ;
- les alkyles de 1 à 20, et de préférence de 1 à 6 atomes de carbone, et notamment les groupes méthyle, éthyle, n-propyle ou iso-propyle ; lesdits groupes alkyle pouvant être non substitués ou substitués par un groupe -OR₀ avec R₀ qui représente un atome d'hydrogène ou un alkyle de 1 à 20, et de préférence de 1 à 6, atomes de carbone, un aryle ou un groupe aralkyle de 7 à 18 atomes de carbone, et notamment les groupes méthoxy, éthoxy, n-propoxy ou iso-propoxy et phénoxy ;
- les groupes cycloalkyle, et notamment les groupe cyclopentyle, cyclohexyle et cycloheptyle ; lesdits groupes alkyle pouvant être non substitués ou substitués par un groupe -OR₀ avec R₀ qui représente un atome d'hydrogène ou un alkyle de 1 à 20, et de préférence de 1 à 6, atomes de carbone, un aryle ou un groupe aralkyle de 7 à 18 atomes de carbone, et notamment les groupes méthoxy, éthoxy, n-propoxy ou iso-propoxy et phénoxy ;
- les groupes phényle et naphtyle ;
- les groupes nitro (-NO₂), carboxyl (-COOH),
- les groupes -OR₀ avec R₀ qui représente un atome d'hydrogène ou un alkyle de 1 à 20, et de préférence de 1 à 6, atomes de carbone, un aryle ou un groupe aralkyle de 7 à 18 atomes de carbone, et notamment les groupes méthoxy, éthoxy, n-propoxy ou iso-propoxy et phénoxy ;
- les groupes amino -NR₀R'₀ avec R₀ et R'₀, identiques ou différents, qui représentent un atome d'hydrogène ou un alkyle de 1 à 20, et de préférence de 1 à 6, atomes de carbone, un aryle ou un groupe aralkyle de 7 à 18 atomes de carbone, et notamment les groupes -NH₂, -NHMe, -NMe₂, - NHEt, -NEt₂.

5. **-** Complexe selon l'une des revendications précédentes **caractérisé en ce que** R₁ représente un groupe un groupe alkyle de 1 à 6 atomes de carbone, un groupe alkyle de 1 à 6 atomes de carbone interrompu par un groupe amino, un groupe cycloalkyle de 3 à 7 atomes de carbone, lesdits groupes alkyle et cycloalkyle pouvant être non substitués ou susbtitués par un groupe -OR₀ avec R₀ qui représente un atome d'hydrogène ou un alkyle de 1 à 20, et de préférence de 1 à 6, atomes de carbone, un aryle ou un groupe aralkyle de 7 à 18 atomes de carbone, et notamment un groupe méthoxy, éthoxy, n-propoxy, iso-propoxy ou phénoxy.

6. **-** Complexe selon l'une des revendications 1 à 4 **caractérisé en ce que** R₁ représente :
- un groupe phényle, un groupe 4-fluorophényle ou 2-fluorophényle, un groupe phényle substitué par un ou plusieurs groupes alkyle ou cycloalkyle porteurs ou non d'un groupe -OR₀ avec R₀ qui représente un atome d'hydrogène ou un alkyle de 1 à 20, et de préférence de 1 à 6, atomes de carbone, un aryle ou un groupe aralkyle de 7 à 18 atomes de carbone, et notamment un groupe méthoxy, éthoxy, n-propoxy, iso-propoxy ou phénoxy,
- un groupe 2-pyridyle ou un groupe 2-pyridyle substitué par un ou plusieurs groupes alkyle ou cycloalkyle porteurs ou non d'un groupe -OR₀ avec R₀ qui représente un atome d'hydrogène ou un alkyle de 1 à 20, et de préférence de 1 à 6, atomes de carbone, un aryle ou un groupe aralkyle de 7 à 18 atomes de carbone, et notamment un groupe méthoxy, éthoxy, n-propoxy, iso-propoxy ou phénoxy, ou
- un groupe 2-thiophényle ou un groupe 2-thiophényle substitué par un ou plusieurs groupes alkyle ou cycloalkyle porteurs ou non d'un groupe -OR₀ avec R₀ qui représente un atome d'hydrogène ou un alkyle de 1 à 20, et de préférence de 1 à 6, atomes de carbone, un aryle ou un groupe aralkyle de 7 à 18 atomes de carbone, et notamment un groupe méthoxy, éthoxy, n-propoxy, iso-propoxy ou phénoxy.

7. **-** Complexe selon l'une des revendications 1 à 4 **caractérisé en ce que** R₁ représente :
- un groupe phényle substitué en position 2 ou 4 par -NO₂ ou par un groupe alcoxy de 1 à 20, et de préférence de 1 à 6, atomes de carbone ou un groupe cycloalcoxy de préférence de 3 à 7 atomes de carbone, les partie alkyle et cycloalkyle étant porteuse ou non d'un groupe -OR₀ avec R₀ qui représente un atome d'hydrogène ou un alkyle de 1 à 20, et de préférence de 1 à 6, atomes de carbone, un aryle ou un groupe aralkyle de 7 à 18 atomes de carbone, et notamment un groupe méthoxy, éthoxy, n-propoxy, iso-propoxy ou phénoxy ; ou
- un groupe 4-imidazolyle ou un groupe 4-imidazolyle substitué par un ou plusieurs groupes alkyle ou cycloalkyle porteurs ou non d'un groupe -OR₀ avec R₀ qui représente un atome d'hydrogène ou un alkyle de 1 à 20, et de préférence de 1 à 6, atomes de carbone, un aryle ou un groupe aralkyle de 7 à 18 atomes de carbone, et notamment un groupe méthoxy, éthoxy, n-propoxy, iso-propoxy ou phénoxy.

8. **-** Complexe selon l'une des revendications précédentes **caractérisé en ce que** Ln est choisi parmi Y, Gd, Eu, Tb, Yb, Er, Tm, Pr, Ho et Nd.

9. **-** Matériau formé d'un substrat, fonctionnalisé en surface par greffage covalent de plusieurs complexes (A) identiques ou différents selon l'une des revendications précédentes, le greffage covalent étant réalisé selon une liaison Si-O par l'intermédiaire d'un des atomes de silicium présent dans Si(ORₐ)₃ d'au moins un des substituants Z₁ tels que définis pour (A) dans les revendications précédentes.

10. **-** Matériau selon la revendication 9 sous la forme d'un objet massif, d'un film ou de nanoparticules, notamment sous la forme d'une poudre.

11. **-** Matériau selon la revendication 10 **caractérisé en ce que** le substrat est constitué d'un oxyde d'un élément M et la liaison covalente entre le complexe (A) est le substrat est Si-O-M, l'élément M étant de préférence le silicium, ou un métal, par exemple Cu ou Al.

12. **-** Matériau selon la revendication 10 ou 11 **caractérisé en ce que** le substrat est fonctionnalisé en surface par greffage covalent de différents complexes (A) qui diffèrent par la nature de l'ion Ln³⁺ piégé ou par la nature du substituant R₁.

13. **-** Matériau selon l'une des revendications 10 à 12 précédentes **caractérisé en ce que** les complexes (A) forment un revêtement avec création de ponts -Si-O-Si entre complexes.

14. **-** Matériau selon l'une des revendications 10 à 13 **caractérisé en ce que** les complexes greffés en surface du substrat sont recouverts d'une couche de silice, éventuellement fonctionnalisée, par exemple par des fonctions -NH₂, -C≡CH, -COOH, -N₃ ou -OH.
